Europäisches Patentamt

**European Patent Office**

**Office européen des brevets**

(11) Publication number: **0 094 216**
A1

(12) # EUROPEAN PATENT APPLICATION

(21) Application number: **83302560.4**

(22) Date of filing: **06.05.83**

(51) Int. Cl.³: **G 01 N 33/54,** B 01 L 3/00,
B 01 L 9/06

(30) Priority: **07.05.82 US 376023**

(43) Date of publication of application: **16.11.83**
**Bulletin 83/46**

(84) Designated Contracting States: **AT BE CH DE FR GB IT
LI LU NL SE**

(71) Applicant: **CENTOCOR, INC., 244 Great Valley Parkway,
Malvern, PA 19355 (US)**

(72) Inventor: **Hevey, Richard C., 302 Weatherstone Drive,
Paoli Pennsylvania 19301 (US)**

(74) Representative: **Harvey, David Gareth et al, Graham
Watt & Co. Riverhead, Sevenoaks Kent TN13 2BN (GB)**

(54) **Diagnostic test apparatus for use in an immunoassay and method employing the apparatus.**

(57) An improved test tube rack (48) for receiving beads employed in an immunoassay is used with a matching sample tray (32), the tray and rack featuring aligning pins and holes (52, 53 and 34, 35) so that the test tubes (42) are juxtaposed with associated sample wells (32) when the rack (48) is assembled to the tray (30). The test tubes (42) are a push-fit in holes (50) in the rack (48) and are larger at their mouths than their bases; the test tubes (42) and the wells (32) interfit tightly to avoid leakage of liquid when the assembled tray and rack are inverted to deposit the beads in the test tubes. The rack and the tray are correspondingly numbered for identification of the sample wells (32) with the test tubes (42).

"DIAGNOSTIC TEST APPARATUS FOR USE IN AN IMMUNOASSAY
AND METHOD EMPLOYING THE APPARATUS"

This invention is in the field of immunology
and particularly relates to a diagnostic test apparatus
for immunoassay determination of diseases associated
with antigens and antibodies.  The invention also
concerns a method useful in immunoassays.

Conventional radioimmunoassay is employed by
hospitals and clinical laboratories for the early
detection of hepatitis, amongst other diseases.  In
testing for viral hepatitis, polystyrene balls  which
have been coated with test samples and radiolabeled
antibodies are placed in a receptacle tray.  The
receptacle tray consists of a series of wells for
receiving the polystyrene balls, which are also known
as beads.  Upon completion of a reaction, the beads
are washed free of unbound radiolabeled antibody and
are measured in a suitable manner for radioactive
decay.

Typically, test samples consist of antigens
which may bind radiolabeled antibody.  The higher
the amount of antigen, the larger the amount of
radiolabeled antibody that binds and therefore
the higher the radioactive count that is made.

In order to measure radioactivity, however, the beads must be transferred to a test tube or counting tube for insertion into the well of a radioactive counter. This can be achieved by picking up the beads one at a time and depositing them in test tubes. This process is time consuming and may lead to error if the beads are not transferred with diligence and then appropriately labeled. Various testing systems currently available attempt to correct this problem and minimize the chance of error.

A bead transfer system is currently marketed by Abbott Laboratories and is discussed in their patent, U.S.-PS 3,932,141. In the Abbott kit or system, plastics test tubes are arranged in a matrix and are held in a cardboard box by a paper top with small holes. The cardboard box is inverted over a plastics receptacle tray supplied with the kit. After the tubes are aligned with the receptacles, the entire assembly is inverted and the polystyrene beads fall into the tubes from the receptacles or wells therefor in the tray. The paper top is then torn from the cardboard box and the tubes may then be removed for counting.

Although this is an improvement over picking out the styrene balls one by one, it has a number of problems. The receptacle tray may easily be placed incorrectly upon the box. Also, there is a gap between the test tubes and the tray so when the beads are transferred it is relatively easy for a bead or liquid occasionally to miss a tube. In addition, all the tubes are themselves identical, and, if more than one tube is removed from the box, they may easily be confused.

Another kit system is currently available from North American Biological, Inc. This kit makes use of a receptacle tray and cardboard box much like the Abbott system. An addition is made, however, in that plastics pins must be inserted in the box for the box with the test tubes to sit properly upon the receptacle tray before inversion. These pins may only be inserted in one manner so that the box is not easily misaligned with the tray. It should, however, be noted that in this as in the Abbott system, the test tubes are identical and do not seat with the receptacle wells accommodating the polystyrene beads. Therefore, it is still possible for the beads occasionally to miss the test tubes and for test tubes to be confused after counting has begun. In neither of the above systems are the test tubes visible in their entirety since they sit in a cardboard box. Therefore any mistakes or problems in bead transfer would not be noticed without removing the tubes individually from the box. Furthermore, defective or broken tubes will not be noted and a valuable test may have to be repeated.

In both of the above-mentioned systems, numbers are printed on the side of the box by which each tube may be identified. Such a numerical system may be misread easily when picking out a centrally-located test tube.

The invention is directed to improvements in the construction and use of bead transfer devices of the kind discussed above.

According to the present invention, there is provided a diagnostic test apparatus suitable for use in an immunoassay, comprising a receptacle tray having wells for receiving samples and a rack of test tubes to be juxtaposed with the wells to receive samples therefrom upon inversion of the juxtaposed tray and rack, characterised in that the tray and rack are made of resilient material and have aligning means in the form of an interfitting pilot pin and pilot hole, the test tubes each being generally cylindrically shaped, composed of resilient material and having a varying outside diameter to form a friction fit with the rack when pressed into test tube receiving holes therein, the tray and rack being marked with correlating indicia.

The invention also provides a method of transferring radio immunoassay beads characterized, after binding test samples and radio labeled antibodies in a receptacle tray having a plurality of identified receptacle wells, by placing upon said receptacle wells a test tube rack with fitted test tubes in a specific identified orientation; pressing said test tubes onto said receptacle wells to form a tight mating fit; inverting the receptacle tray and test tube rack as an assembly to cause transfer of radio immunoassay beads to the test tubes; removing the receptacle tray from the assembly; and releasing the test tubes from their fit with the test tube rack.

. The description which now follows is given by way of non-limiting example.

The invention in its preferred embodiments utilizes a receptacle tray similar to those commonly used but with two significant differences. Each of the receiving wells for the antibody coated polystyrene ball or bead is· individually labeled with a number. In addition, the tray is equipped with two holes located at its corners for aligning

- 6 -       0094216

a test tube rack therewith.  The test tube rack includes two pins for insertion into these holes of the receptacle tray.

The tray preferably has a matrix of twenty e.g. twenty five sample wells.

The test tube rack consists of a matching array of friction fitted test tubes in resilient material.  Each of the test tubes is individually labeled on the tray in a manner to match the labeling of the receptacle tray.  The test tubes themselves are of resilient material and increase in diameter from the base of the tube to the top of the tube.  This allows for tubes to be inserted into the test tube rack but will not allow them to pass through the rack; rather the hole in the rack forms a seat for the test tube.  When the test tubes are pressed down into the rack, the rack may be inverted over the receptacle tray without the tubes falling out.  The pins are positioned so that the test tube rack may only be put over the receptacle tray in one way.  At this point, the test tubes are pushed down onto the receiving wells.

The receiving wells of the receptacle tray are slightly tapered and of the correct diameter to mate as male pieces with the mouths of the test tubes which serve as female pieces. A solid fit is therefore formed between the receiving wells and the test tubes which guards against leakage of liquid and/or polystyrene missing the test tubes during transfer. For transfer, the assembly is inverted so that the test tubes are upright with their mouths facing upwards. The receptacle tray can then be removed leaving the test tube rack in the upright position and labeled identically to the wells in the receptacle tray. If the test tube rack is then pushed downwards by pressing on tabs moulded into the rack, the tubes are loosened in the rack and may be easily removed. Tubular columns depending from the test tube holes form the test tube rack base and protect the test tubes from damage.

In addition to the above-mentioned identification system, the tubes have indicia upon them to make confusion of samples more difficult. In the preferred embodiment, the tubes are of five different colours. The five different colours are arranged in a consecutive sequence so that no two tubes of the same colour follow one another numerically. This colour coding makes it much more difficult accidentally to switch the tubes for, in a 25 tube matrix, there are only five places for a tube of a particular colour, whereas, if the tubes were unmarked, and two or three tubes were removed from the test tube rack, they may be replaced in any hole without any mistake being apparent.

In an alternative embodiment of the invention, an automatic test tube plug identification and insertion system is used. After the test tubes are released from their friction fit with the test tube rack, a flexible sheet of plastic material is aligned by the pins and placed upon the tubes. A plunger tool is then used to push precut plugs from the flexible sheet into the test tubes. The plugs are pre-numbered to match the matrix numbers disposed upon the test tube rack. The flexible sheet is then removed minus the cutout plugs, and the plugged test tubes are then ready for analysis.

The invention will now be described in further detail by way of example only with reference to the accompanying drawings, in which:

Figure 1 is a perspective view of a receptacle tray,

Figure 2 is a perspective view of a matching test tube rack and showing two test tubes,

Figure 3 is a perspective view of a test tube plug board, and

Figure 4 is a perspective view of a test tube plug insertion tool.

In the accompanying drawings like reference characters refer to the like parts throughout the different views.  The drawings are not necessarily to scale, emphasis instead being placed upon illustrating the principles of the invention.

The component pieces of the embodiment illustrated are best constructed of translucent, e.g. transparent resilient material which allows for strength, translucency and friction fit assembly.

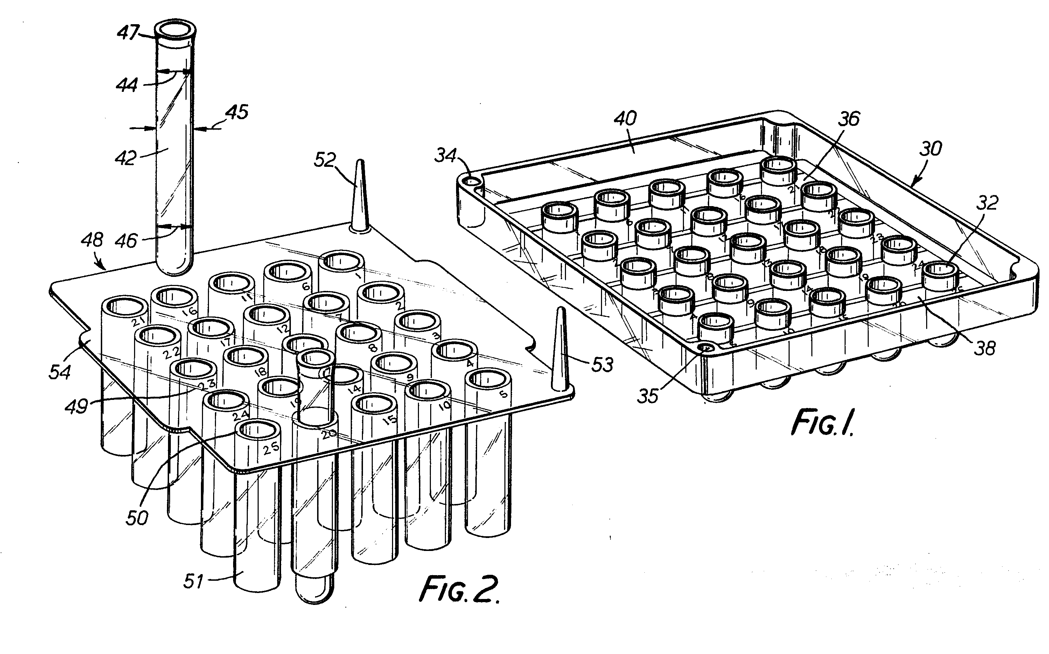

Figure 1 is a perspective view of a receptacle tray 30.  The receptacle tray has twenty-five wells 32, for receipt of test beads, arranged in a 5 x 5 matrix.  Each of the wells 32 is marked individually with numerals 36 so that test samples will not be confused.  Also of interest, the receptacle tray has two holes 34, 35 at adjacent corners of the tray for the alignment of the tray with the test tube rack to be described.

Underneath the wells 32 a stiffening member 38 adds strength to the receptacle wells and prevents breakage.  A stiffening member 38 connects five wells in each row in the matrix.  Also visible in this view is retaining wall 40.  The retaining

- 10 -                    0094216

wall 40 provides for structural strength and ease of
handling.  It surrounds the matrix of wells on all
four sides and projects above the height of the
wells.

In Figure 2 may be seen two of the twenty-five
test tubes 42 that fit into the test tube rack.  The
test tubes 42 are each generally cylindrical; however,
the diameter at the base 46 is smaller than that at
the upper portion 44.  The inner diameter 44 at the
upper portion is slightly larger than the outer
diameter of the top of each receptacle well 32.  In
addition, there is a reinforcing band 47 at the
opening of the test tube.  The increased diameter
and the reinforcing band 47 are provided so that the
test tube may be pushed down onto a receptacle well
to form a tight seal.  The purpose of this is to
ensure complete transfer from the receptacle well of
the test bead without allowing accidental
misplacement of the bead or any leakage of
liquid to an adjacent well.

Figure 2 shows in perspective view the test
tube rack 48 in which the test tubes 42 are
inserted.  The rack 48 is desirably made from a
transparent material.  The test tube holes 50 are
slightly smaller in diameter than the maximum outer
diameter 45 of the test tubes so they cannot pass through the rack, but rather can be retained by a friction fit in their holes.

The test tube rack has two aligning pins 52, 53 to be inserted into the bead receptacle tray 30. Having two pins 52, 53 in the manner shown, the test tube rack may only be mated with the receptacle tray in one orientation. This orientation is such that the numeric markings 49 of the test tube rack will match the numeric markings 36 of the receptacle tray.

In Figure 2, test tube columns 51 can be seen projecting down from the holes 50. The columns serve as both protection for the test tubes and form a base for the test tube rack.

A review of the use of the receptacle tray and test tube rack is now in order. Twenty-five test tubes 42 are inserted into the twenty-five holes 50. Each test tube is pressed down into the hole and because of their increasing diameter, they form a friction fit with the test tube holes 50. Once the test tubes 42 have been inserted into the test tube rack 48 and forced down relatively tightly, the test tube rack is inverted. The inverted rack is placed over the receptacle tray 30. When the test tube rack is seated in position, the test tubes are individually pressed down onto the receptacle wells 32 to form tight seals therewith. When this has been done, the entire assembly is once again turned over. Test beads and any liquid in the wells are thus transferred into the associated test tubes. After the assembly is inverted, the receptacle tray is removed from the test tube rack. Tabs 54 on the test tube rack 48, Figure 2, are then pushed down to free the test tubes from their friction fit in their holes 50. The rack base is formed by the bottoms of the columns 51 aligned with the test tube holes 50. The test tubes are now free to be easily removed from the test tube rack 48 as the need arises.

It should be noted that the markings on the test tube rack are identical to the markings' of the receptacle tray so that no test samples will be confused. In addition, the test tubes have their own indicia included. In this embodiment, the test tubes are of five different colours. In this way, colour coded test tubes may not be easily confused. For example, all the test tubes in one row at right angles to the numerical sequence starting with 1 might be blue and all the test tubes in the row starting with 2, red, and so on. In this case, two contiguous test tubes, each located in a different row, could not be incorrectly replaced on the test tube tray without an obvious colour misplacement.

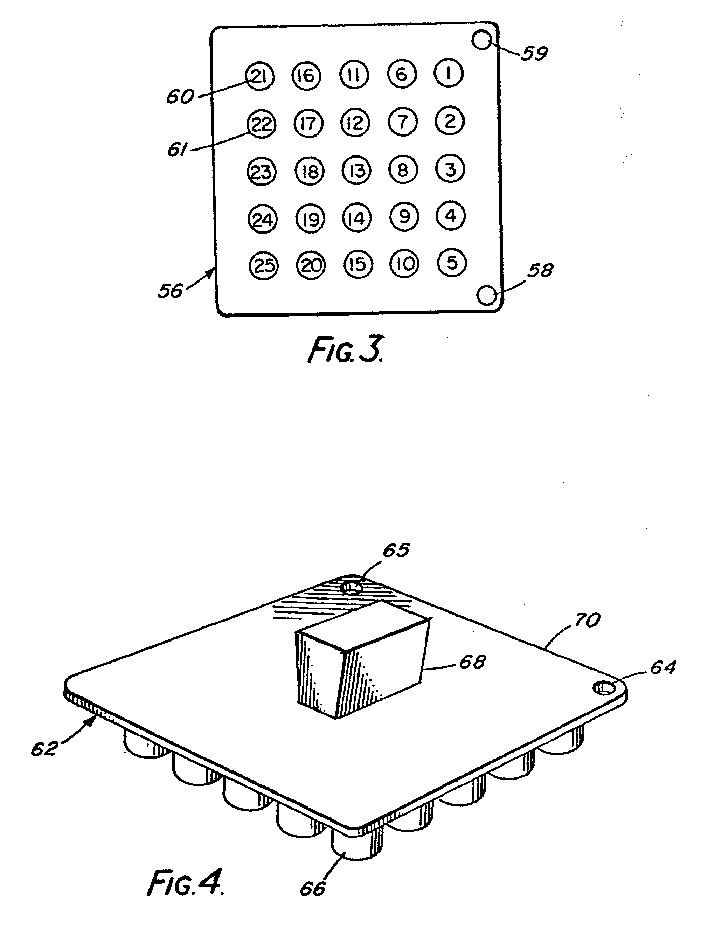

In Figure 3 is shown a test tube plug board 56 which is placed over the test tubes once they have been released from the friction fit with the test tube rack 48. The board has two holes 58, 59 to engage with the two pins 52, 53 on the test tube rack, for alignment purposes. Numerical indicia, 60, 61, etc., face upwards when the plug board 56 has been properly positioned. In this way, such indicia are visible and match the numerical indicia of the test tube rack 48. The plugs 61 are arranged in a 5 x 5 matrix matching the test tubes and have been die cut from the plug board but not severed entirely therefrom. The plug board can be made from polyurethane.

In Figure 4 is shown a test tube plug insertion tool 62 which pushes the plugs from the plug board 56 into the test tubes 42. The insertion tool 62 is constructed from a plastics sheet 70 to which the handle 68 and various plungers 66 are attached. The plungers 66 are in a matrix matching the matrix of test tubes and plugs previously described. Holes 64, 65 in the tool are positioned to align with the pins 52, 53 which protrude above the plug board. As the tool is pushed down by the handle 68, the plungers 66 force the plugs from the plug board into the test tubes.

After the plugs have been inserted into the test tubes, the insertion tool 62 and the plug board 56 are lifted from the pins.  The pins 52, 53 themselves may then be removed from the test tube rack by snipping or breaking.  What remains are twenty-five test tubes in a matrix each labeled by means of its plug 60, 61, etc., its colour and the number 49 on the test tube rack itself.  In this way the combined system leaves very little room for confusion or mistake in the analysis of the immunoassay beads.

While the invention has been particularly shown and described with reference to preferred embodiments thereof, it will be understood by those skilled in the art that various changes in form and detail may be made.  For example, the indicia marking the test tubes may be changed to letters or different colours or various other markings on the test tubes themselves.  Also alternative aligning means, such as a different set of aligning pins and pilot holes, might be devised.  A single pin and hole will suffice if appropriately shaped.

Claims:

1. A diagnostic test apparatus, suitable for use in an immunoassay, comprising a receptacle tray having wells for receiving samples and a rack of test tubes to be juxtaposed with the wells to receive samples therefrom upon inversion of the juxtaposed tray and rack, characterised in that the tray (30) and rack (48) are made of resilient material and have aligning means in the form of an interfitting pilot pin (e.g. 52) and pilot hole (e.g. 34), the test tubes (42) each being generally cylindrically shaped, composed of resilient material and having a varying outside diameter to form a friction fit with the rack (48) when pressed into test tube receiving holes (50) therein, the tray (30) and rack (48) being marked with correlating indicia (36, 49).

2. A diagnostic test apparatus according to claim 1 characterised in that the receptacle tray (30) has a matrix of at least twenty sample wells (32).

3. A diagnostic test apparatus according to claim 1 or claim 2, characterised in that the test tubes (42) have indicia of marking which correlate with the markings on the test tube rack (48).

4. A diagnostic test apparatus according to claim 1, 2 or 3, characterised by the test tubes being colour coded.

5. A diagnostic test apparatus according to claim 1, 2, 3 or 4, characterised by the rack (48) and tray (30) may only be assembled in one orientation.

6. A diagnostic test apparatus according to any of claims 1 to 5, characterised in that the receptacle wells (32) have outer diameters smaller than the inner diameters of the test tubes, so that each receptacle well may be inserted into the mouth of a test tube and form a seal therewith.

7. A diagnostic test apparatus according to any of claims 1 to 6, further characterised by a plunger tool (62) and precut plug board (56) for inserting test tube plugs (60) into several test tubes essentially simultaneously.

8. A diagnostic test apparatus according to claim 7, further characterised by the test tube plugs (60) being marked with indicia matching the markings on the rack (48).

9. A diagnostic test apparatus according to any of claims 1 to 8, characterised by the test tube rack being transparent.

10. A diagnostic test apparatus according to any of claims 1 to 8, characterised by all components thereof being essentially transparent.

11. A diagnostic test apparatus according to any of the preceding claims, for use in immunoassay of antigens and antibodies, characterised in that the test tube rack (48) has indicia marked at each hole (50) and is arranged only to be assembled to the receptacle tray (30) in one orientation, its test tubes being of slight conical shape and composed of resilient material, and further characterised in that the test tubes (42) are dimensioned to fit tightly into the test tube rack (48), each test tube having a largest inner diameter greater than the minimum outer diameter of the receptacle wells so that the test tubes and receptacle wells can form a tight male and female fit of one with the other.

12. A method of transferring radio immunoassay beads characterised, after binding test samples and radio labeled antibodies in a receptacle tray having a plurality of identified receptacle wells, by placing upon said receptacle wells a test tube rack with fitted test tubes in a specific identified orientation; pressing said test tubes onto said receptacle wells to form a tight mating fit;

inverting the receptacle tray and test tube rack as an assembly to cause transfer of radio immunoassay beads to the test tubes; removing the receptacle tray from the assembly; and releasing the test tubes from their fit with the test tube rack.

13. A method according to claim 12, further characterised by inserting simultaneously a plurality of identifying die cut test tube plugs into said test tubes by means of a multiple plug inserting tool.

FIG.1.

FIG.2.

1/2

0094216

Fig.3.

Fig.4.

**European Patent Office**

**EUROPEAN SEARCH REPORT**

Application number

EP 83 30 2560

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | US-A-4 154 795 (A. THORNE) <br> * Whole document * | 1-13 | G 01 N 33/54 <br> B 01 L 3/00 <br> B 01 L 9/06 |
| A | FR-A-2 378 283 (SEARLE & CO.) <br> * Page 1, line 5 - page 3, line 15; Claims 1-4 * | 1-13 | |
| A | GB-A-1 485 729 (ABBOTT LABORATORIES) | | |
| A | GB-A-2 004 768 (ANDREAS HETTICH) <br> * Abstract; figures 1-5; page 1, line 71 - page 2, line 13; claims 1-6 * | 1-13 | |

TECHNICAL FIELDS SEARCHED (Int. Cl. ³)

G 01 N
B 01 L

The present search report has been drawn up for all claims

| Place of search | Date of completion of the search | Examiner |
|---|---|---|
| THE HAGUE | 29-07-1983 | OSBORNE H.H. |